# EUROPÄISCHE PATENTSCHRIFT

(11) **EP 2 433 660 B1**
(45) Veröffentlichungstag und Bekanntmachung des Hinweises auf die Patenterteilung: **30.07.2014**
(21) Anmeldenummer: 11179132.3
(22) Anmeldetag: 29.08.2011
(51) Int. Cl.: A61L 31/02, A61L 31/10, A61L 31/06, A61L 31/14, A61L 27/34, A61L 27/04, A61L 27/54, A61L 31/16

(54) **Beschichtetes Implantat aus einer biokorrodierbaren Magnesiumlegierung**
Coated implant composed of a biocorrodible magnesium alloy
Implant revêtu en alliage de magnésium biocorrodable

(30) Priorität: 28.09.2010 US 387048 P
(43) Veröffentlichungstag der Anmeldung: 28.03.2012
(73) Patentinhaber: Biotronik AG, 8180 Bülach (CH)
(72) Erfinder: Borck, Alexander, 91086 Aurachtal (DE)
(74) Vertreter: Lindner-Vogt, Karin L.

(56) Entgegenhaltungen:
- WO-A2-2006/108065
- WO-A2-2008/092436

## Beschreibung

Die Erfindung betrifft ein beschichtetes Implantat mit einem Grundkörper aus einer biokorrodierbaren Magnesiumlegierung.

### Stand der Technik und Hintergrund der Erfindung

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z.B. Stents), zur Befestigung und temporären Fixierung von Gewebeimplantaten und Gewebstransplantationen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch A-neurysmenstents bekannt, die vorrangig zur Abdichtung des Aneuryrismas dienen. Die Stützfunktion ist zusätzlich gegeben.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder Ti-A16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit biokorrodierbaren Basislegierungen des Magnesiums.

Bekannt ist ferner, dass sich ein höheres Maß an Biokompatibilität erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Beschichtung bereitgestellt werden.

Der Einsatz biokorrodierbarer Magnesiumlegierungen für temporäre Implantate mit filigranen Strukturen ist insbesondere dadurch erschwert, als dass die Degradation des Implantats in vivo sehr rasch verläuft. Um die Korrosionsrate, d. h. die Abbaugeschwindigkeit, zu mindern, sind verschiedene Ansätze in der Diskussion. Zum Einen wird versucht, auf Seiten des Implantatswirkstoffs durch entsprechende Legierungsentwicklung die Degradation zu verlangsamen. Zum Anderen sollen Beschichtungen eine temporäre Hemmung des Abbaus bewirken. Letzterer Ansatz erfordert demnach, dass die Beschichtung für einen reproduzierbaren Zeitraum die Korrosion verhindert oder hemmt, dann jedoch rasch eine vollständige Auflösung des Implantats ermöglicht.

### Zusammenfassung der Erfindung

Ein oder mehrere der zuvor angesprochenen Nachteile des Standes der Technik werden mit Hilfe des erfindungsgemäßen Implantats mit einem Grundkörper aus einer biokorrodierbaren Magnesiumlegierung und einer Beschichtung gelöst oder zumindest gemindert. Die Beschichtung des Implantats umfasst:
(i) eine auf dem Grundkörper aufgetragene Grundschicht aus einem basischen Polymer mit einer Repetiereinheit, die Einheiten der Formeln (1) oder (2) enthält 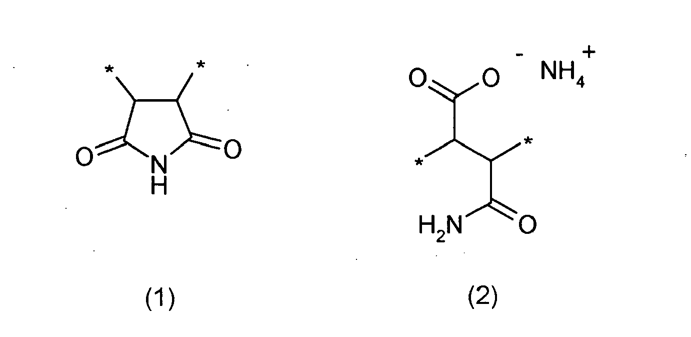 und
(ii) eine polymere Deckschicht, die direkt auf der Grundschicht aufgetragen ist.

Der Erfindung liegt die Erkenntnis zugrunde, dass sich die Korrosion von Implantaten aus biokorrodierbaren Magnesiumlegierungen dadurch zeitlich hinauszögern lässt, dass eine doppellagige polymere Beschichtung aus einer auf dem Grundkörper aufgetragenen Grundschicht und einer diese Grundschicht abdeckenden Deckschicht aufgebracht wird. Das Polymer der Grundschicht weist eine Imidgruppe der Formel (1) oder ein Amid-Ammonium-Salz der Formel (2) als einen basischen Bestandteil seiner Repetiereinheit auf. Durch eine basische Mikroumgebung an der Oberfläche des Grundkörpers wird der Abbau der Magnesiumlegierung verzögert. Die basische Mikroumgebung wird durch das Polymer der Grundschicht erzeugt, das in den Repetiereinheiten die genannte Gruppe aufweist. Durch ein Schichtsystem, bestehend aus einer Lewisbase und einer polymeren Deckschicht, kann das chemische Potential lokal verändert werden.

Vorzugsweise ist das basische Polymer der Grundschicht ein Polymer der Formel (3) oder (4): 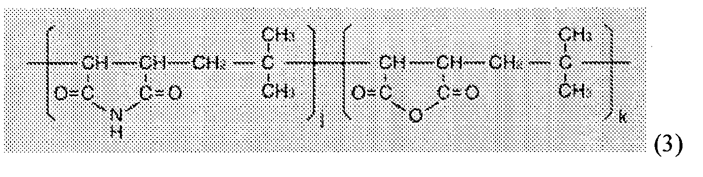 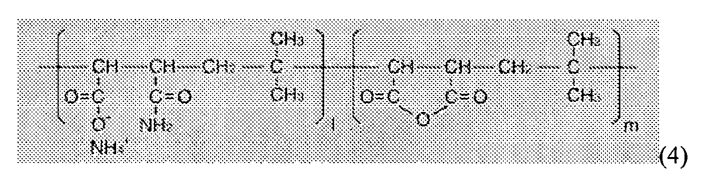 wobei j, k, 1 und m so gewählt sind, dass das Polymer eine Molmasse im Bereich von 50.000 bis 100.000 g/mol, insbesondere 80.000 bis 90.000 g/mol, aufweist. Diese Molmassenbereiche haben sich in der experimentellen Testung als besonders vorteilhaft erwiesen. Vorzugsweise ist j = k und 1 = m. Polymere, die unter Formel (3) (Polyisobutylen-alt-maleimid; CAS-Nr: 89360-06-5) und Formel (4) (CAS-Nr: 52032-17-4) fallen, sind kommerziell erhältlich.

Nach einer weiteren bevorzugten Variante enthält die Deckschicht Polylactid-co-Glycolid (PLGA) oder besteht aus diesem Polymer. Die Deckschicht kann insbesondere einen Wirkstoff enthalten.

Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 100 µm, besonders bevorzugt 300 nm bis 15 µm. Die erfindungsgemäße Beschichtung kann direkt auf die Implantatoberfläche aufgetragen werden oder es sind weitere Zwischenschichten vorgesehen; so kann gegebenenfalls der Grundkörper des Implantats eine anorganische Grundschicht aufweisen, die die Haftung der erfindungsgemäßen Beschichtung verbessert. Auf die erfindungsgemäße Beschichtung können weitere Schichten aufgetragen werden (zum Beispiel so genannte top coat - Schichten). Verfahren zur Beschichtung von Implantaten sind dem Fachmann bekannt.

Als biokorrodierbar im Sinne der Erfindung werden Legierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Unter Magnesiumlegierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C und pH 7,38 gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die ― bei Einsatz des Werkstoffs in einem Bauteil - zu einer Beeinträchtigung der Funktion des Bauteils führt. Ein Korrosionssystem besteht hier aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Salinität, Volumen-Oberflächen-Verhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden Transport- und Diffusionsprozessen. Bei Magnesiumlegierungen ist in der Regel eine allmähliche Alkalisierung der Doppelschicht zu beobachten. Fremdstoffablagerungen, Verunreinigungen und Korrosionsprodukte beeinflussen den Korrosionsprozess. Die Vorgänge bei der Korrosion, insbesondere der Geschwindigkeitsbestimmende Schritt, sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme. Dies gilt insbesondere für Stents, welche bei der Implantation lokal hohen plastischen Verformungen ausgesetzt sind. Konventionelle Ansätze mit starren korrosionshemmenden Schichten sind unter derartigen Voraussetzungen ungeeignet.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird ein im Sinne der Erfindung beschichtetes Implantat zur Herabsetzung der Korrosionsrate führen. Die erfindungsgemäße korrosionshemmende Beschichtung kann im Laufe der Zeit selbst abgebaut werden bzw. kann die von ihr abgedeckten Bereiche des Implantats nur noch in einem immer geringeren Umfang schützen. Daher ist der Verlauf der Korrosionsrate für das gesamte Implantat nicht linear. Vielmehr ergibt sich zu Beginn der einsetzenden korrosiven Prozesse eine relativ niedrige Korrosionsrate, die im Laufe der Zeit ansteigt. Dieses Verhalten wird als temporäre Herabsetzung der Korrosionsrate im Sinne der Erfindung verstanden und zeichnet die korrosionshemmende Beschichtung aus. Im Falle von Koronarstents soll die mechanische Integrität der Struktur über einen Zeitraum von drei bis sechs Monaten nach Implantation aufrechterhalten werden.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten. Vorzugsweise ist das Implantat ein Stent.

Die Deckschicht kann einen oder mehrere Wirkstoffe enthalten, der nach der Implantation freigesetzt wird. Der Wirkstoff kann in das polymere Material eingebettet sein.

Unter Wirkstoff im erfindungsgemäßen Sinne wird ein Arzneistoff mit pharmazeutischer Wirkung verstanden, der im menschlichen oder tierischen Körper zur Heilung, Linderung, Verhütung oder Erkennung von Krankheiten dient. Wirkstoffe umfassen insbesondere Paclitaxel, Sirolimus und dessen Derivate. Insbesondere sind Wirkstoffe vorteilhaft, die auf mTOR wirken, sowie RAS Inhibitoren, insbesondere solche, die die Adhäsion von RAS an die Zellmembran verhindern.

### Kurzbeschreibung der Figuren

Die Erfindung wird nachfolgend anhand eines Ausführungsbeispiels und der dazugehörigen Figuren näher erläutert. Es zeigen:
- Fig. 1: eine schematische Darstellung des Aufbaus der erfindungsgemäßen Beschichtung; und
- Fig. 2: eine lichtmikroskopische Aufnahme eines beschichteten Stents nach Testung.

### Detaillierte Beschreibung der Erfindung

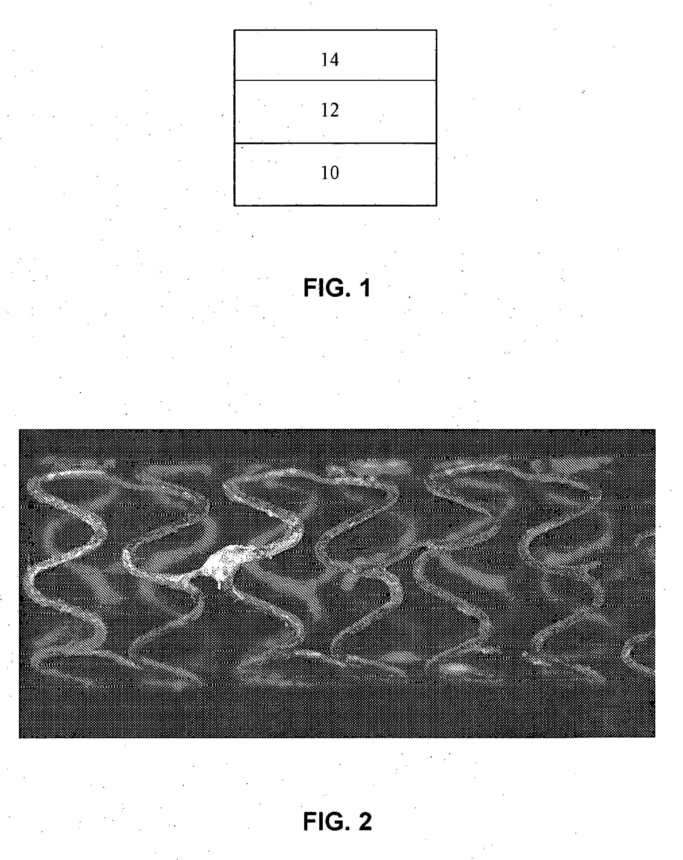

Fig. 1 illustriert stark schematisiert den Aufbau einer erfindungsgemäßen Beschichtung. Ein Grundkörper 10 aus einer biokorrodierbaren Magnesiumlegierung wird von einer polymeren Grundschicht 12 bedeckt. Direkt auf der Grundschicht 12 wird eine polymere Deckschicht 14 aufgetragen.

Die Erfindung wird nachfolgend anhand eines Ausführungsbeispiels näher erläutert.

### Ausführungsbeispiel

Ein Stent aus einer biokorrodierbaren Magnesiumlegierung wird in eine Lösung von Polyisobutylen-alt-maleimid (2 g in 40 ml DMF) für 45 min. bei Raumtemperatur getaucht. Nach der Entnahme und Trockentupfen wird der Stent auf einem fusselfreien Tuch an der Luft für 30 min. getrocknet. Anschließend wird der Stent in eine Lösung von PLGA (Handelsname Resomer LG 502; 0,2 gin 40 ml CHCl₃) für 40 Sek. bei Raumtemperatur getaucht. Es folgt erneutes Abtupfen und Trocknen für 1 h bei 75°C im Ofen.

### Stents wurden wie folgt getestet:

Beschichtete und unbeschichtete Stents aus einer biokorrodierbaren Magnesiumlegierung wurden auf einem Ballonkatheter montiert. Für das Korrosionsexperiment wurden die Stents mit dem Ballon in 200 ml einer PBS Lösung (100 mM; pH 7,38) getaucht und in dieser Lösung dilatiert und freigesetzt. Die Lösung wurde gerührt und auf Raumtemperatur gehalten.

Figur 2 ist eine lichtmikroskopische Aufnahme eines erfindungsgemäß beschichteten Stents nach Testung. Es ist deutlich die noch erhaltene Struktur des Stents erkennbar. Deutlich ist der metallische Glanz zu erkennen, der zeigt, dass der Grundkörper zwar angegriffen aber noch vorhanden ist.

Zu Vergleichszwecken wurden Stents ohne Beschichtung, nur mit Grundschicht, nur mit Deckschicht und einem homogenen Gemisch aus den polymeren Materialien der Grund- und Deckschicht demselben Korrosionstest unterzogen. Alle diese Stents haben sich unter gleichen Bedingungen komplett aufgelöst; selbst Fragmente konnten nicht mehr geborgen und analysiert werden. Sowohl das Polyisobutylen-alt-maleimid als auch das PLGA allein zeigten demnach keinen die Korrosion hemmenden Einfluss.

Durch das Polymer der Grundschicht, das in jeder zweiten Repetiereinheit eine Imidgruppe aufweist, wird eine basische Mikroumgebung erzeugt. In einer ähnlichen Betrachtungsweise wird die Elektronendichte in der Grundschicht zum Substrat hin verschoben, wodurch das Substrat "edler" wird und seine Korrosionsgeschwindigkeit verlangsamt wird. Dieser Effekt wird beobachtet, wenn die Grundschicht Elektronenpaardonatoren, insbesondere Elemente der 5. und/oder 6. Hauptgruppe des Periodensystems enthält. Die Elemente dieser Hauptgruppen, insbesondere die Elemente Schwefel, Stickstoff, Phosphor und Sauerstoff, verfügen über ungepaarte Elektronenpaare, die leicht verschiebbar sind. Diese Ergebnisse bestätigen, dass durch die basische Mikroumgebung das chemische Potential lokal beeinflusst werden kann. Die Maleinimidgruppe wirkt als Elektronendonor (Lewisbase). Der basische Einfluss muss - das zeigen die Experimente - durch eine weitere Polymerschicht geschützt werden.

## Patentansprüche

1. Implantat mit einem Grundkörper aus einer biokorrodierbaren Magnesiumlegierung und einer Beschichtung umfassend:
(i) eine auf dem Grundkörper aufgetragene Grundschicht aus einem basischen Polymer mit einer Repetiereinheit, die Einheiten der Formeln (1) oder (2) enthält 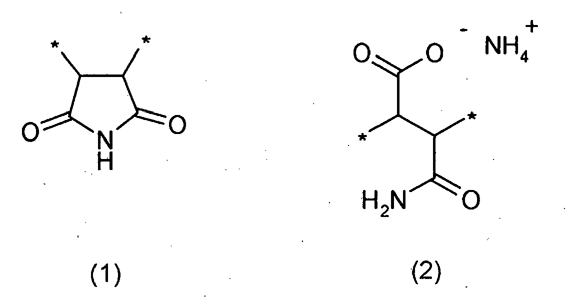 und
(ii) eine polymere Deckschicht, die direkt auf der Grundschicht aufgetragen ist.

2. Implantat nach Anspruch 1, bei dem das basische Polymer der Grundschicht ein Polymer der Formel (3) oder (4) ist 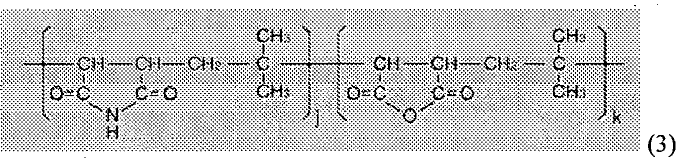 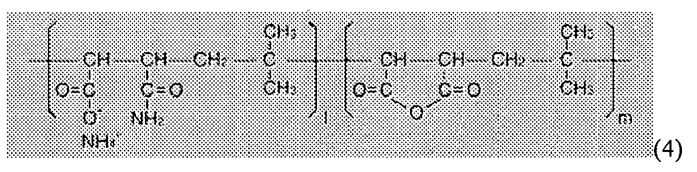 wobei j, k, 1 und m so gewählt sind, dass das Polymer eine Molmasse im Bereich von 50.000 bis 100.000 g/mol aufweist.

3. Implantat nach einem der vorhergehenden Ansprüche, bei dem die Deckschicht Polylactid-co-Glycolid (PLGA) enthält.

4. Implantat nach einem der vorhergehenden Ansprüche, bei dem die Deckschicht einen Wirkstoff enthält.

5. Implantat nach Anspruch 4, bei dem der Wirkstoff Paclitaxel, Sirolimus oder ein Derivat von Sirolimus ist.

6. Implantat nach einem der vorhergehenden Ansprüche, bei dem das Implantat ein Stent ist.

7. Implantat nach einem der vorhergehenden Ansprüche bei der die Grundschicht einen Elektronenpaardonator enthält.

8. Implantat nach Anspruch 7, bei der die Grundschicht als Elektronenpaardonator ein oder mehrere Elemente der 5. Hauptgruppe des Periodensystems, insbesondere Stickstoff oder Phosphor, enthält.

9. Implantat nach Anspruch 7, bei der die Grundschicht Elektronenpaardonator ein oder mehrere Elemente der 6. Hauptgruppe des Periodensystems, insbesondere Schwefel oder Sauerstoff, enthält.

## Claims

1. An implant having a base body composed of a biocorrodible magnesium alloy and a coating comprising:
(i) a base layer, which is applied to the base body, composed of a basic polymer having a repeater unit that contains units of formula (1) or (2) 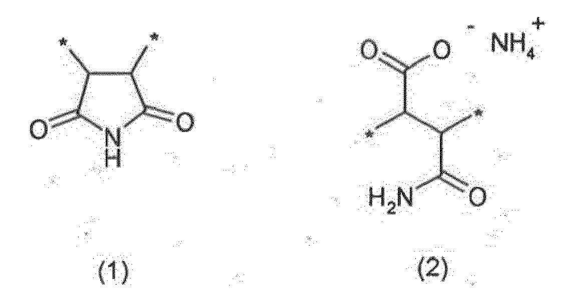 and
(ii) a polymeric cover layer that is applied directly to the base layer.

2. The implant according to claim 1, wherein the basic polymer of the base layer is a polymer having the formula (3) or (4) 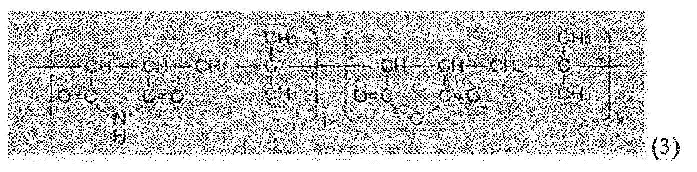 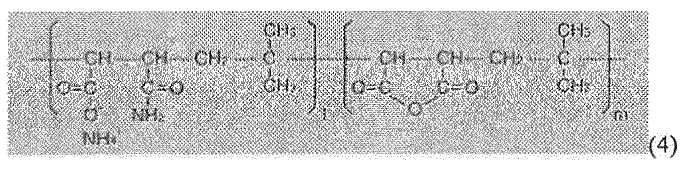 wherein j, k, l and m are selected such that the polymer has a molar mass in the range 50,000 to 100,000 g/mol.

3. The implant according to one of the preceding claims, wherein the cover layer contains poly(lactide-co-glycolide) (PLGA).

4. The implant according to one of the preceding claims, wherein the cover layer contains an active ingredient.

5. The implant according to claim 4, wherein the active ingredient is paclitaxel, sirolimus, or a derivative of sirolimus.

6. The implant according to one of the preceding claims, wherein the implant is a stent.

7. The implant according to one of the preceding claims, wherein the base layer contains an electron-pair donor.

8. The implant according to claim 7, wherein the base layer, as the electron-pair donor, contains one or more elements of the 5^{th} main group of the periodic table, in particular nitrogen or phosphorous.

9. The implant according to claim 7, wherein the base layer, as the electron-pair donor, contains one or more elements of the 6^{th} main group of the periodic table, in particular sulphur or oxygen.

## Revendications

1. Implant avec un corps de base constitué d'un alliage de magnésium bio-corrodable et d'un revêtement comprenant :
(i) une couche de base appliquée sur le corps de base et constituée d'un polymère basique avec une unité de répétition contenant des unités des formules (1) ou (2) 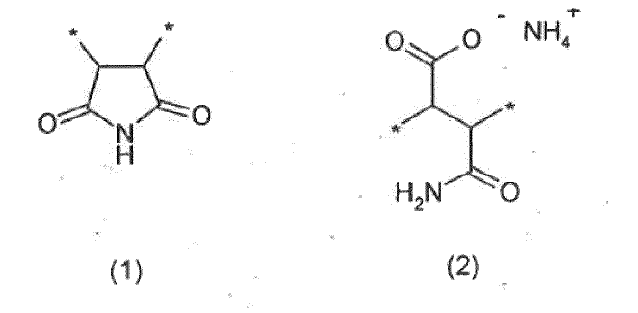 et
(ii) une couche de couverture polymère appliquée directement sur la couche de base.

2. Implant selon la revendication 1, dans lequel le polymère basique de la couche de base est un polymère de la formule (3) ou (4) 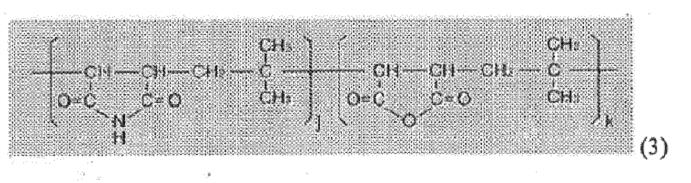 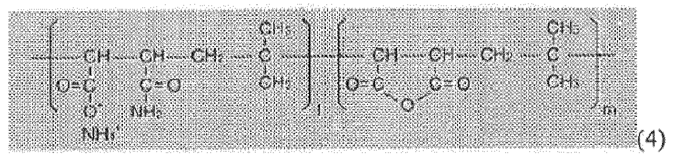 dans lesquelles j, k, 1 et m sont choisis de telle sorte que le polymère présente une masse moléculaire comprise entre 50.000 et 100.000 g/mol.

3. Implant selon l'une des revendications précédentes, dans lequel la couche de couverture contient un polylactide-co-glycolide (PLGA).

4. Implant selon l'une des revendications précédentes, dans lequel la couche de couverture contient une substance active.

5. Implant selon la revendication 4, dans lequel la substance active est un paclitaxel, un sirolimus ou un dérivé de sirolimus.

6. Implant selon l'une des revendications précédentes, dans lequel l'implant est un stent.

7. Implant selon l'une des revendications précédentes, dans lequel la couche de base contient un donneur à paire d'électrons.

8. Implant selon la revendication 7, dans lequel la couche de base en tant que donneur à paire d'électrons contient un ou plusieurs éléments du 5^{e} groupe principal du système périodique, en particulier de l'azote ou du phosphore.

9. Implant selon la revendication 7, dans lequel la couche de base donneuse à paire d'électrons contient un ou plusieurs éléments du 6^{e} groupe principal du système périodique, en particulier du soufre ou de l'oxygène.
